# EUROPEAN PATENT SPECIFICATION

(11) **EP 3 099 231 B1**
(45) Date of publication and mention of the grant of the patent: **06.04.2022**
(21) Application number: 15701917.5
(22) Date of filing: 15.01.2015
(51) Int. Cl.: A61B 5/1455, A61B 5/00, A61B 5/11

(54) **SEIZURE DETECTION BASED ON WORK LEVEL EXCURSION**
ANFALLSERKENNUNG BASIEREND AUF EINER ABWEICHUNG AUF ARBEITSEBENE
DÉTECTION DE CRISE BASÉE SUR UNE EXCURSION DE NIVEAU DE TRAVAIL

(30) Priority: 30.01.2014 US 201414169006
(43) Date of publication of application: 07.12.2016
(73) Proprietor: Flint Hills Scientific, LLC, Lawrence, KS 66044 (US)
(72) Inventor: OSORIO, Ivan, Lawrence, Kansas 66044 (US)
(74) Representative: Grünecker Patent- und Rechtsanwälte PartG mbB
(86) International application number: PCT/US2015/011604
(87) International publication number: WO 2015/116402

(56) References cited:
- WO-A1-2013/171599
- US-A- 4 883 063
- US-A1- 2010 261 982
- US-A1- 2012 029 390
- US-A1- 2012 271 372
- US-A1- 2012 277 605

## Description

### FIELD OF THE INVENTION

This disclosure relates to a medical device capable of detecting epileptic seizures.

### PRIOR ART

US 2012/0029390 A1 discloses seizure detection, wherein cardiac cycle information is used as well as location information and motion information in order to detect seizures. The dynamic seizure threshold is defined in dependence on the location and motion information.

US 2012/0271372 A1 discloses a method for detecting and reacting to seizures, wherein cardiac data is used in order to obtain a cardiac index, which is to be compared with extreme values. A severity index is determined in order to adapt the therapy accordingly.

US 2012/277605 A1 discloses detection of a neurological event based on a comparison of a difference in heart rates detected at different times with some dynamic threshold. When the threshold is exceeded, a warning is issued and the time of seizure is recorded. A relatively high threshold for identifying a seizure event when the patient is engaged in a sedentary activity and a relatively low threshold for identifying a seizure event when the patient is engaged in a strenuous activity are determined.

### SUMMARY OF THE INVENTION

It is provided a medical device, comprising:
a body data module configured to receive body data;
a work level data module configured to determine a first patient work level during a first time
   period, and a second patient work level during a second time period;
a work level excursion module configured to:
   determine whether said second work level exceeds said first work level by an amount greater than a first work level excursion threshold; and
   detect a work level excursion, in particular, a pathological work level excursion, based upon a determination that said second work level exceeds said first work level by an amount greater than said first work level excursion threshold, wherein said work level excursion threshold is based at least in part on one or more of a time of day, an environmental condition, an indicator of said patient's hydration status, said patient's body weight and height, said patient's body mass index, said patient's gender, or said patient's age; and
   a controller to perform at least one of warning of said work level excursion, providing a therapy for said work level excursion, or logging an occurrence of said work level excursion., in response to detecting said work level excursion.

A device in accordance with the invention is defined in claim 1.

Any method is not part of the invention.

### DESCRIPTION OF THE DRAWINGS

The disclosure may be understood by reference to the following description taken in conjunction with the accompanying drawings, in which like reference numerals identify like elements, and in which:
Figure 1 shows a schematic diagram of a medical device system, in accordance with some embodiments of the present disclosure;
Figure 2 shows a schematic diagram of portions of a medical device system, in accordance with some embodiments of the present disclosure;
Figure 3 shows a schematic diagram of a work level data module, according to some embodiments of the present disclosure;
Figure 4 shows a variation of work level range over the course of a day, according to some embodiments of the present disclosure;
Figure 5 shows a schematic diagram of a patient state data unit, according to some embodiments of the present disclosure;
Figure 6 shows a flowchart depiction of a method, according to some embodiments of the present disclosure; and
Figure 7A shows a flowchart depiction of some steps of the method of Figure 7, according to some embodiments of the present disclosure;
Figure 7B shows a flowchart depiction of some steps of the method of Figure 7, according to some embodiments of the present disclosure;
Figure 8 shows a flowchart depiction of a method, according to some embodiments of the present disclosure;
Figure 9A shows a flowchart depiction of a method of measuring the work level of the patient's body or a part thereof, according to some embodiments of the present disclosure;
Figure 9B shows a flowchart depiction of a method of measuring the work level of the patient's body or a part thereof, according to some embodiments of the present disclosure;
Figure 10A qualitatively shows the typical correspondence between activity level and work level for convulsive seizures, according to some embodiments of the present disclosure;
Figure 10B qualitatively shows a typical correspondence between non-seizure activity level and non-seizure work level, according to some embodiments of the present disclosure;
Figure 10C qualitatively shows a typical lack of correspondence between non-seizure activity level and seizure work level, for both partial and convulsive seizures, according to some embodiments of the present disclosure; and
Figure 11 shows a flowchart depiction of a method of detecting a seizure, according to some embodiments of the present disclosure.

While the disclosure is susceptible to various modifications and alternative forms, specific embodiments thereof have been shown by way of example in the drawings and are herein described in detail. It should be understood, however, that the description herein of specific embodiments is not intended to limit the disclosure to the particular forms disclosed, but on the contrary, the scope of the invention is defined by the claims.

### DETAILED DESCRIPTION OF SPECIFIC EMBODIMENTS

Illustrative embodiments of the disclosure are described herein. For clarity, not all features of an actual implementation are described. In the development of any actual embodiment, numerous implementation-specific decisions must be made to achieve design-specific goals, which will vary from one implementation to another. Such a development effort, while possibly complex and time-consuming, would nevertheless be a routine undertaking for persons of ordinary skill in the art having the benefit of this disclosure.

Some embodiments disclosed herein provide for performing a convulsive seizure detection based upon a work level of a patient. Body signal(s) from a patient, such as arterio-venous differences in oxygen, may be acquired during a first time period. Based at least partially upon the body signal(s), a work level of the patient for the first time period is determined. A determination can then be made as to whether the work level exceeds an extreme work level threshold or some other non-extreme reference level value. If the work level exceeds an extreme threshold, a determination that a convulsive seizure has occurred may be made. Further, a confirmation test to confirm the convulsive seizure may be performed.

"Work level" herein refers to the energy consumption of the patient's body or a part thereof *(e.g.,* the brain). Energy consumption may, in some embodiments, be measured by differences in oxygen saturation, concentration, or pressure between arterial and venous locations. In some embodiments of this disclosure, work level may be determined using measures of kinetic activity (force, amplitude, velocity, direction, and duration and rate of muscle contractions) or autonomic activity such as heart rate. "Work level" in this disclosure does *not* refer exclusively to the patient's physical activity. Physical activity may be only one of multiple physiological events or states leading to energy consumption by the patient's body or a part thereof. "Work level" may also refer to the physics definition, W = F x d. The application of force over distance may be only one of multiple actions by a patient leading to energy consumption by the patient's body or a part thereof.

One of the meanings of extreme work level herein is a work level that cannot be accomplished volitionally. Extreme work levels may exceed the maximal volitional work level that may be performed by the patient per unit time, and/or may exceed the total duration of a work that could be sustained volitionally by a patient. Convulsive (generalized tonic or clonic or tonic-clonic) seizures are associated with a work level (determined using metabolic, kinetic or other signals) that is extreme.

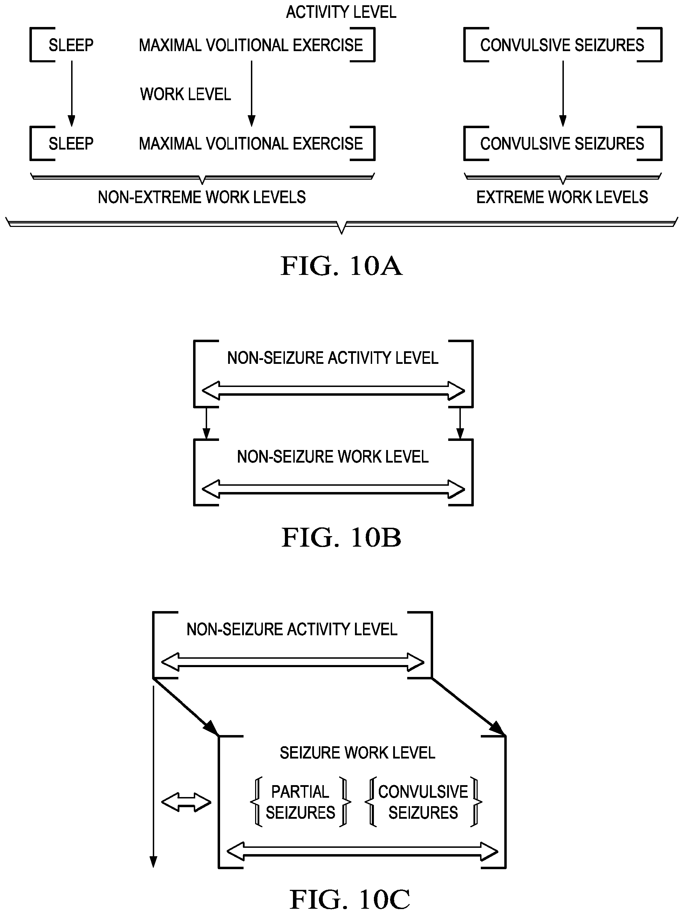

Figure 10A illustrates, in qualitative fashion, typical correspondence or commensurateness between ranges (minimum to maximum) of activity and work levels for non-seizure conditions, e.g., from sleep up to maximal volitional exercise. It further illustrates that while the activity and work levels for convulsive seizures are much greater than for non-seizures, these levels are still commensurate.

Figure 10B illustrates, in qualitative fashion, typical commensurateness between activity and work levels for non-seizure or interictal periods for a typical patient.

Figure 10C depicts, in qualitative fashion, typical differences between non-seizure activity level and seizure work level. Notice that while the work level for partial seizures is generally within the non-seizure activity level range, it is incommensurate with the current (e.g., at the time(s) the change in work level occurs) non-seizure activity level. The work level of epileptic convulsive seizures is well above the non-seizure activity level range. On the other hand, the work level of generalized atonic seizures and partial seizures manifesting with motionlessness or with bradycardia may be below the non-seizure activity range and/or incommensurate with the current non-seizure activity level. Thus, "incommensurateness" as used herein may be observable by a work level outside an expected work level range, above an upper work level threshold, and/or below a lower work level threshold.

In another embodiment of the present disclosure, a pathological state of a patient may be detected based upon an excursion of a work level of the patient. The excursion may relate to an increase in a work level during a second time period compared to a work level during a first time period. Body signal(s) from a patient may be acquired during a first time period. Based at least partially upon the body signal(s), a first work level of the patient for the first time period is determined. Body signal(s) from a patient may be acquired during a second time period. Based at least partially upon the body signal(s), a second work level of the patient for the second time period is determined. The first and second work levels are compared and a determination is made whether the second work level exceeds the first work level by a work level excursion threshold.

In one embodiment, if the second work level exceeds the first work level by a certain quantity that may be set as a threshold, a pathological state may be deemed to have occurred. In general, a threshold may be set in a relative or absolute manner dependent upon the context and clinical application of the situation. In another embodiment, if the second work level does not exceed the first work level but said work level is incommensurate with the on-going activity levels *(e.g.,* too high or too low a work level relative to a work level expected for the patient's activity level), a pathological state may be deemed to have occurred.

In response to detecting the pathological state, a responsive action may be taken, wherein the responsive action may include providing a warning (*e.g.*, locally (at the site where the patient is located at the time of the convulsion) and/or remotely (to an EMT, a doctor's office, a nurse's station, etc.), providing a therapy, and/or logging the occurrence of the pathological event and its characteristic (*e.g.,* severity, prevailing conditions at date/time of occurrence, etc.). The environmental factors surrounding the occurrence of the pathological event may also be logged and taken into account into the determination of the pathological event. In an alternative embodiment, the above analyses may be performed by using a moving window to acquire body data signals. In one embodiment, the work level excursion threshold may be determined based upon at least one of the body signal acquired during the first time period or the work level during the first time period.

Determination of the value of the extreme work level threshold would allow classification of seizures into convulsive and non-convulsive (e.g. partial): Work levels that reach or exceed the extreme threshold correspond to convulsions (*e.g.,* generalized tonic, tonic-clonic, clonic-tonic-clonic or myoclonic) and those associated with work levels that are incommensurate with corresponding activity levels but remain below the extreme threshold, correspond to partial seizures. That is, work level excursions or degree of incommensurateness between activity level and work level may be used in one or more embodiments in this disclosure, to detect and quantify seizures and to classify them as epileptic or non-epileptic (in the case of convulsive seizures) and the epileptic seizures as convulsive or non-convulsive (*e.g.,* partial). Moreover, these two observables may be used to distinguish inter-ictal from the ictal and post-ictal states.

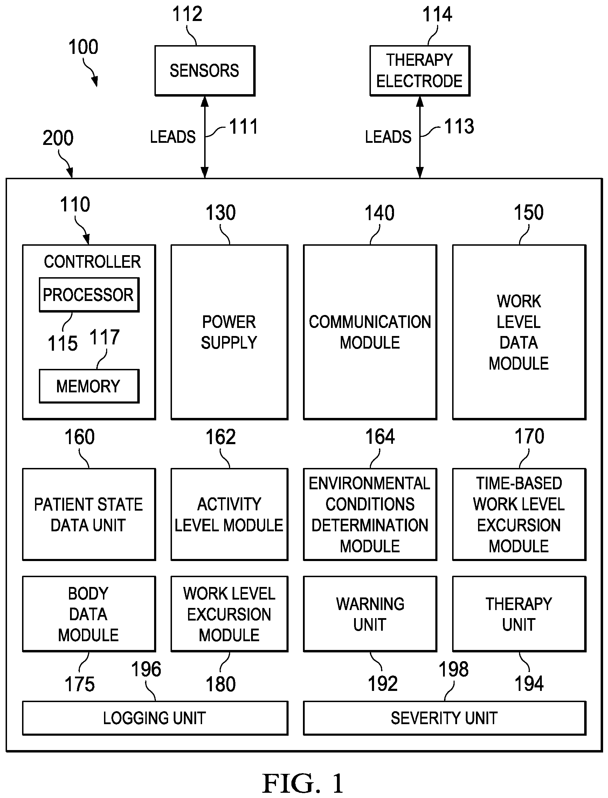

Figure 1 shows a schematic representation of a medical device system, according to some embodiments of the present disclosure. The medical device system 100 may comprise a medical device 200, body data sensor(s) 112, and lead(s) 111 coupling the sensor(s) 112 to the medical device 200. In one embodiment, body data sensor(s) 112 may each be configured to collect data from a patient relating to a time series of body data values. In one embodiment, the body data may be selected from heart rate, blood pressure, respiratory rate, dermal activity, oxygen saturation, or end-tidal CO₂ among others.
"Oxygen saturation" is used herein to encompass measures of oxygen in the patient's blood. Such measures include O₂ concentration and O₂ partial pressure, among others. In yet other embodiments, glucose or ATP consumption or production of certain byproducts of metabolism, such as CO₂ or lactic acid, may be used to determine work level in the brain, other body parts (*e.g.,* muscles), or the body as a whole.
Various components of the medical device 200, such as controller 110, processor 115, memory 117, power supply 130, communication module 140, warning unit 192, therapy unit 194, logging unit 196, and severity unit 198 have been described in other patent applications assigned to Flint Hills Scientific, LLC or Cyberonics, Inc., such as, USSN 12/896,525, filed October 1, 2010; USSN 13/288,886, filed November 3, 2011; USSN 13/449,166, filed April 17, 2012; and USSN 13/678,339, filed November 15, 2012.
The medical device 200 may comprise at least one therapy delivery source (*e.g.,* electrode or catheter) 114, configured to deliver an electrical stimulation or some other form of therapy (*e.g.,* a drug) to a tissue of the patient, such as a neural tissue of the patient. An exemplary electrical stimulation therapy is vagus nerve stimulation therapy, such as that provided by implantable VNS devices commercially available from Cyberonics, Inc. Alternatively or in addition, pharmacological, thermal, or other therapies may be used in this disclosure.
The medical device 200 may comprise a body data module 175 configured to receive body data from the at least one sensor(s) 112.
The medical device 200 may comprise a patient's state data unit 160 configured to determine a patient's state based upon one or more of the body data, the time of day, or an activity level history of the patient, among others. A patient's state may be selected from one or more of asleep or awake, resting or active, etc.
The medical device 200 comprises an activity level module 162 configured to determine the patient's activity level based at least in part on one or more of the body data, the time of day, or an activity level history of the patient. The patient activity level differs from patient state by being more short-term in focus and more immediately (in the temporal sense) derived from its inputs. For example, a patient in the awake state may be motionless or active ranging from slow walking to vigorous exercise. These activity levels may change within seconds/instantaneously and their determination is far more susceptible to rapid changes than is the case for a patient that may be continuously in an awake state for 12-16 hours per day. That said, there may occur circumstances wherein the patient activity level and the patient state provide nearly the same information, e.g., when the patient is asleep.

The medical device 200 may comprise and environmental conditions determination module 164 configured to determine one or more environmental conditions (*e.g.,* time of day, ambient temperature, altitude, ambient humidity, barometric pressure, luminance, etc.). One or more of the determined environmental conditions may be provided to other component(s) of the medical device 200, *e.g.,* the patient state data unit 160, the activity level module 162, etc., to allow determinations of patient state, activity level, etc. to be based at least in part on the one or more determined environmental conditions.

The medical device 200 comprises a work level data module 150 configured to determine a patient's work level. For example, the work level data module 150 is configured to determine a first work level of the patient during a first time period, and the patient's second work level during a second time period.

The medical device 200 may comprise a time-based work level excursion module 170 configured to determine a work level expected for a patient at a given time of day, week, month, season, or year. In some embodiments, the time-based work level excursion module 170 may determine the work level expected for a patient based at least in part on at least one of body data or patient state, in addition to time of day, etc.

The medical device 200 may comprise a work level excursion module 180 configured to determine whether a work level is undergoing a work level excursion indicative of a seizure. For example, the work level excursion module 180 may determine if a work level exceeds an extreme work level threshold. In some embodiments, the work level excursion module 180 may determine an extreme work level threshold, such as at a value of three standard deviations above the patient's mean or median work level. This mean may be determined over any time scale *(e.g.,* 1-10sec.; 11sec-1hr; up to 24 hr or more than 24 hr), up to all available data for the patient. In some embodiments, the extreme work level threshold may be set at an elevated level (*e.g.,* more than three standard deviations) or at a lowered level *(e.g.,* less than three standard deviations) from a particular body data state for the patient, such as moderate or strenuous exercise. Other statistical markers (*e.g.,* percentiles) may be used instead of, or in addition, to the mean or median.

For another example, in some embodiments, the work level excursion module 180 may determine the work level expected for a patient based on at least one of body data, time of day, or patient state. In light of a determined expected work level, the work level excursion module 180 may determine if a second work level exceeds a first work level by greater than an amount of a first work level excursion threshold. The first work level excursion threshold may be prespecified and fixed, or adaptive or variable based on factors such as those given elsewhere. For yet another example, the work level excursion module 180 determines if an observed work level is incommensurate with the patient's activity level. The patient's activity level is determined by activity level module 162 described above.

A work level excursion relative to a first work level excursion threshold and/or a range expected for a patient's activity level may be considered an indication of a pathological state, such as a partial epileptic seizure, or a convulsive epileptic seizure.

An extreme work level excursion (beyond an extreme work level threshold) may be considered an indication of a convulsive epileptic seizure.

If a seizure is indicated by a work level excursion, in one embodiment, the controller 110 may be configured to perform at least one responsive action, in response to a determination that said work level excursion is indicative of a seizure, wherein said responsive action is selected from determining said occurrence of said seizure, or implementing a confirmation test to confirm said occurrence of said seizure.

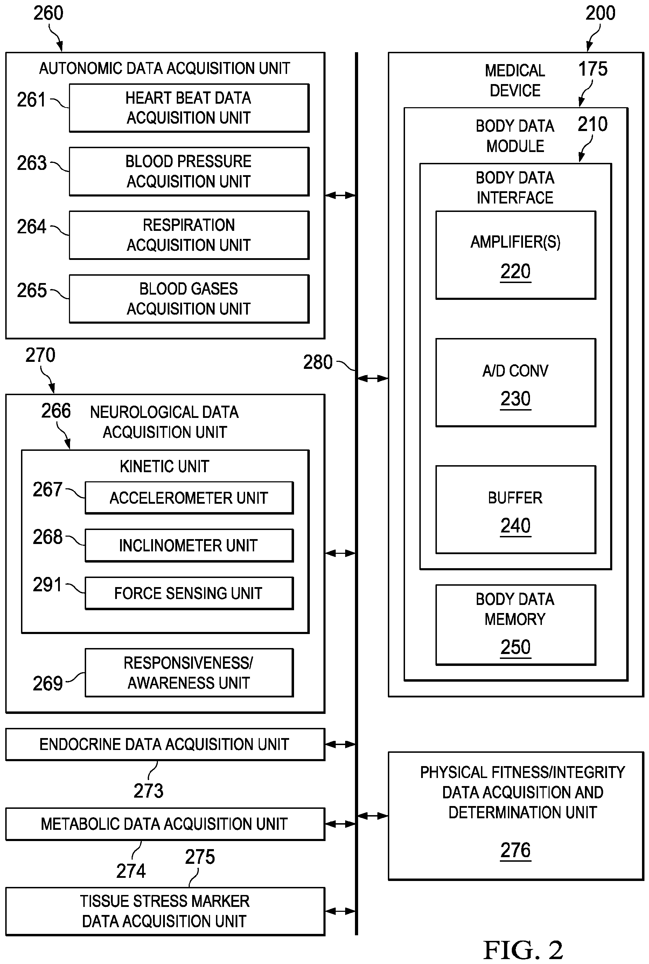

Figure 2 shows a schematic representation of components of the medical device system, particularly, body data module 175 of medical device 200, as well as an autonomic data acquisition unit 260, a neurological data acquisition unit 270, an endocrine data acquisition unit 273, a metabolic data acquisition unit 274, a tissue stress marker data acquisition unit 275, and a physical fitness/integrity data acquisition unit 276. In other, non-depicted embodiments, the medical device system may comprise none, one, or some of the data acquisition units 260-276. More information regarding multiple body data types, data collection thereof, and use thereof in epileptic event detection may be found in other patent applications assigned to Flint Hills Scientific, LLC or Cyberonics, Inc., such as, USSN 12/896,525, filed October 1, 2010, now U.S. Pat. No. 8,337,404, issued December 25, 2012; USSN 13/098,262, filed April 29, 2011; USSN 13/288,886, filed November 3, 2011; USSN 13/554,367, filed July 20, 2012; USSN 13/554,694, filed July 20, 2012; USSN 13/559,116, filed July 26, 2012; and USSN 13/598,339, filed August 29, 2012.
Of interest in Figure 2 is force sensing unit 291 configured to determine an amount of force generated by part or all of a patient's body.
Also of interest in Figure 2 is blood gases acquisition unit 265. In some embodiments, blood gases acquisition unit 265 may be configured to detect blood oxygen saturation, concentration, or pressure at one or more points in a patient's vasculature, (*e.g.,* carotid artery, jugular vein, superior vena cava, inferior vena cava, a left ventricle of the heart, right ventricle of the heart, aorta, branch of the aorta, or a sub-branch of the aorta, among others). In alternative embodiments, blood gases acquisition unit 265 may be configured to detect blood oxygen saturation, concentration, or pressure in other blood vessels and/or organ target sites of the patient's body. In some embodiments, blood gases unit 265 may comprise an arterio-venous oxygen difference determination unit 2741 configured to determine an arterio-venous oxygen difference, and an oxygen consumption determination unit 2742 configured to determine an oxygen consumption from the arterio-venous oxygen difference. In yet other embodiments, CO₂ pressures or concentrations may be measured at one or more body locations.

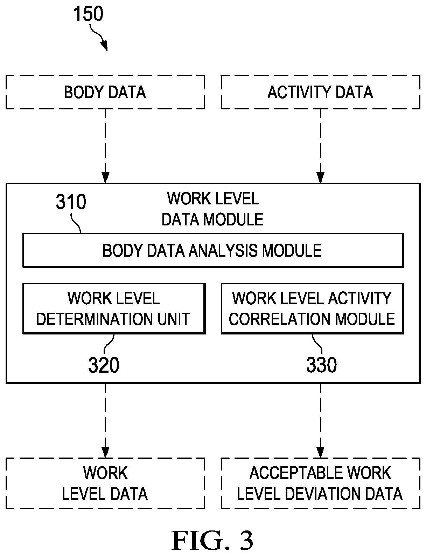

Figure 3 shows a work level data module 150 *(e.g.,* such as that in Figure 1) in more detail. The work level data module 150 may receive one or both of body data and activity data from the patient. The work level data module 150 may comprise a body data analysis module 310 configured to analyze received body data for use by other components of the work level data module 150 or other modules or units of medical device 200.
The work level data module 150 may comprise a work level determination unit 320 configured to determine a work level of a patient from one or more of the received body data or the received activity data. In one embodiment, the work level determination unit 320 may be configured to determine a work level of a patient from body data related to oxygen consumption. Alternatively or in addition, the work level determination unit 320 may be configured to determine a work level of a patient from data related to kinetic activity, cognitive activity, emotional activity, or other patient activity.

The work level data module 150 may comprise a work level-activity correlation module 330 configured to determine a correlation between the work level determined by work level determination unit 320 and certain received body signals data. This correlation may be useful as a check on the determination of the patient's work level by work level determination unit 320 (*e.g.,* is the determined work level expected or within non-pathological limits based on the current activity?) and/or may be informative regarding excursions of the patient's work level from that expected based on the activity. The work level-activity correlation module 330 may provide information regarding acceptable work level deviations. In addition, a work-level correlation with activity level may be either a positive or negative correlation or may have a strong or weak association. Further, a work correlation evaluation with an activity level may show no correlative value (*i.e.,* a zero correlation).

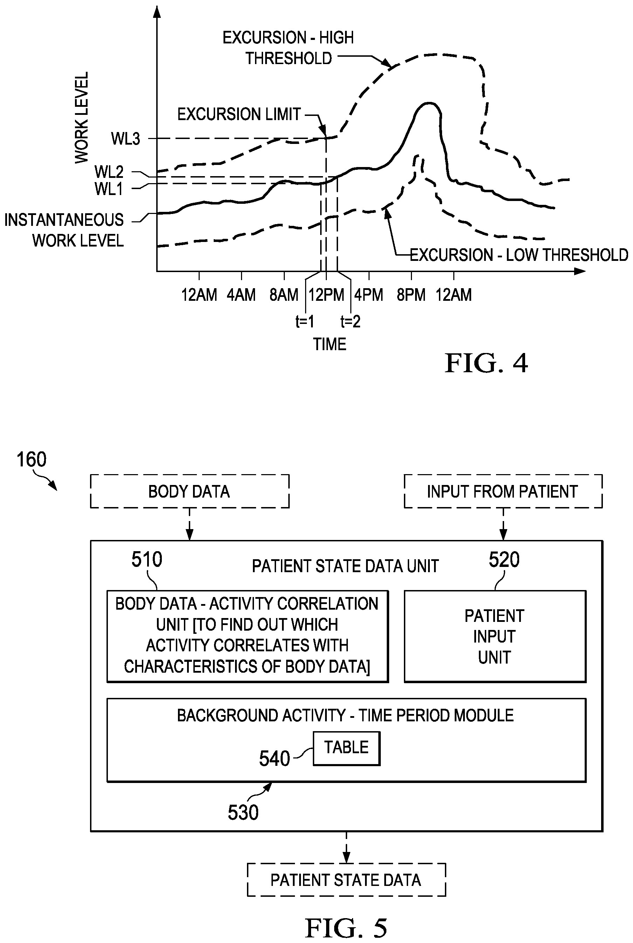

The work level data module 150 may output one or more of work level data or acceptable work level deviation data commensurate with the activity level, among others. Figure 4 shows a typical variation of work level over time of day for an exemplary patient. Generally, an instantaneous body work level (WL) may be at its lowest during sleep, moderate during most waking hours, and high during certain waking hours, e.g., during aerobic exercise, such as the hypothetical patient of Figure 4 is performing at about 9 pm.

As instantaneous work level increases, the thresholds for a work level excursion indicative of a partial or non-convulsive seizure may vary as well. The work level excursion may be a magnitude excursion, a rate of change excursion, or both. Figure 5 shows a patient state data unit 160 in more detail. The patient state data unit 160 may receive body data and/or patient input (*e.g.,* manual input). The patient state data unit 160 may comprise a body data - activity correlation unit 510 configured to find which activity may be correlated with which body data characteristics or properties. For example, if the body data is kinetic data and indicative of long, rhythmic strides *(e.g.,* the patient is jogging or running) this activity may be correlated with certain heart rate, oxygen consumption or tidal respiratory volume data.

Generally, measurements of body work level may be useful for detection of epileptic convulsive seizures. Measurements of brain oxygen consumption may be useful for detection of both convulsive and partial seizures, for distinguishing between convulsive and partial seizures, and for distinguishing unilateral from bilateral partial seizures. A correlation or commensurateness of autonomic and/or other body signal with work level may also be useful to detect partial seizures.

The patient state data unit 160 may comprise a patient input unit 520 configured to receive patient manual input regarding the patient's activity or other parameters.

The patient state data unit 160 may comprise a background activity - time period module 530 configured to determine a background activity level at or over a given time period. The determination made by the background activity - time period module 530 may comprise looking up relevant background activity and/or time period data stored in a table 540. The background activity level may be useful for detection of both generalized and partial seizures using O₂ consumption given by arterio-venous differences in oxygen saturation measured at the appropriate anatomical sites.

In some situations, heart rate, heart rhythm, respiratory rate, respiratory rhythm/pattern, etc. may serve as proxies of activity level.

The patient state data unit 160 may output patient state data for use by other modules or units of the medical device 200.

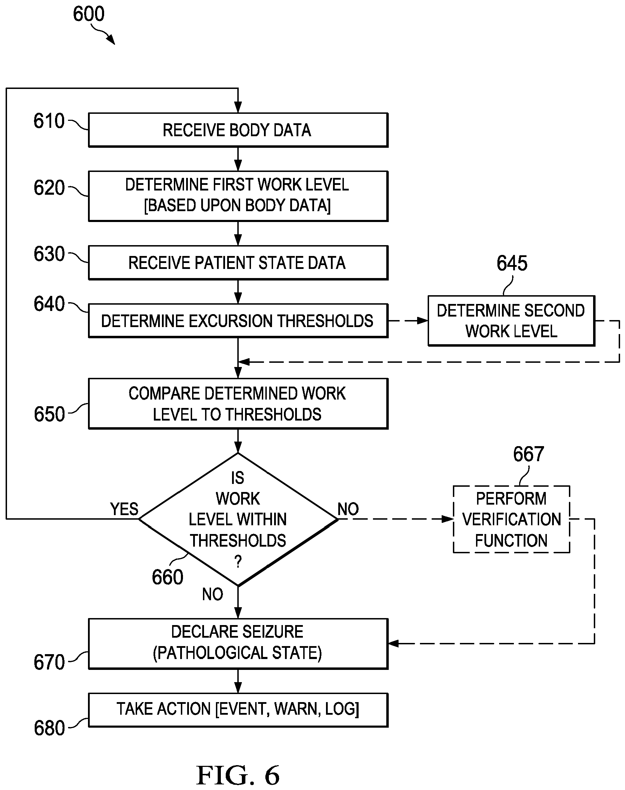

Figure 6 shows a flowchart representation of a method 600 according to some embodiments of the present disclosure. This method may be useful in detecting partial seizures from work level data. Body data, such as those types discussed *supra,* may be received at 610. A first work level may be determined at 620 based at least in part on the body data. Patient state data may be received at 630. In some embodiments, receiving patient state data at 630 is optional and need not be performed. In some situations, the patient state data may be inferred with high confidence, and without the need for receiving it, from the body data and/or other considerations. For example, if the patient has a very low activity level at a time between about midnight and 6:00 am, it may be inferred with high confidence that the patient is asleep.

Based at least in part on one or more of the received body data and the received patient state data, one or more work level excursions may be determined at 640. The work level excursion(s) may be relative to a threshold, outside a non-seizure range, or absolute (*e.g.,* independent of a threshold or range). The non-seizure work level range may be defined at its lower bound by the patient's work level during stage 3 (slow-wave) sleep and at its upper bound by the patient's maximal volitional exertion (*e.g.,* during intense exercise).

Similarly to and associated with the first work level determination at 620, a second work level may be determined at 645.

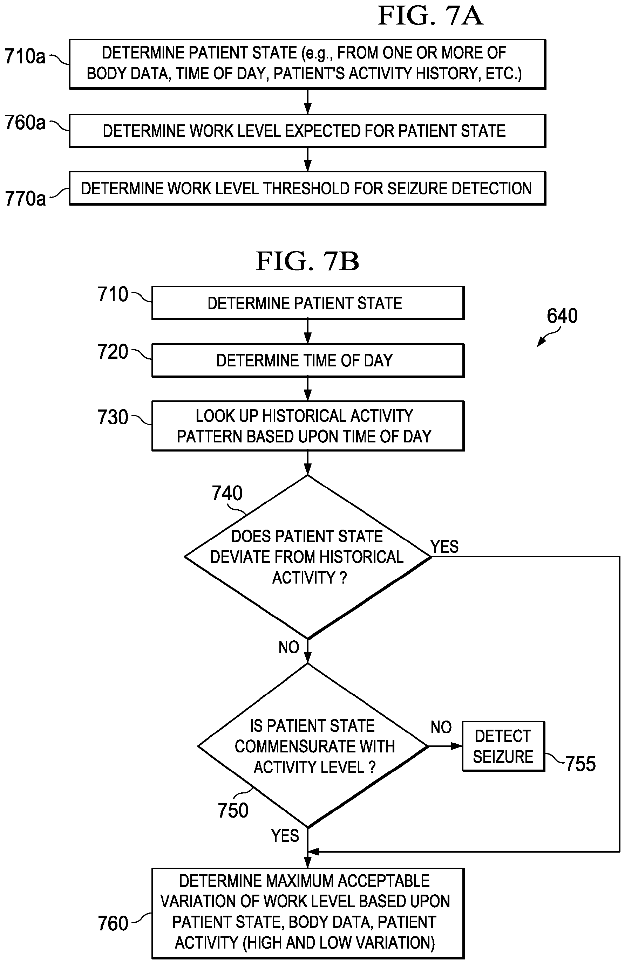

Figure 7A shows one embodiment of determining a work level excursion threshold at 640 in more detail. A patient state may be determined at 710a, based on one or more of body data gathered from the patient, the time of day, the patient's activity history, the patient's current activity, and/or other factors. In light of the determined patient state, an expected work level may be determined at 760a. The expected work level may include statistical measures of central tendency, high percentile value, low percentile value, standard deviation, etc., relating to the expected work level.

From the expected work level, a work level threshold may be determined at 770a. The work level threshold may be determined from a percentile value based on the expected work level *(e.g.,* the threshold may be set at the 95%ile or 99%ile of a statistical set of expected work level values) or a number-of-standard-deviations value based on the expected work level (*e.g.,* the threshold may be set at 2 or 3 standard deviations above a measure of central tendency of expected work level). Regardless of how the threshold is set, the work level threshold may then be used in detecting seizures based on work level excursions above the threshold level.

Figure 7B shows another embodiment of determining a work level excursion threshold at 640 in more detail. A patient state may be determined at 710, and a time of day may be determined at 720. This determination is generally not needed to establish an extreme work level excursion threshold, for reasons discussed *supra.* When activity patterns (by time of day and/activity type and level) exist, the work level corresponding to it with it may be determined at 730. It should be noted that the correlation between work level and activity pattern may be positive or negative, and strong or weak. If the patient state, activity level or type, is determined at 740 to deviate from a pattern or historical activity, then a maximum acceptable variation of work level based on the patient state, body data, and/or patient activity may be determined at 760. If the patient state or activity level or type do not deviate from a pattern or historical activity type or level as determined at 740, then it may be determined at 750 whether the work level pattern or patient state *(e.g.,* asleep, resting awake, etc.) is commensurate (*i.e.,* the observed values are similar to the expected values) with the patient's activity level. If the work level is commensurate with the patient's activity level, then flow may proceed to determining at 760 the maximum acceptable variation of work level, and determining the work level excursion threshold at 765. If the patient state is not commensurate with the patient's activity level, a seizure may be detected at 755 from this observation alone.

In one embodiment, the work level threshold is associated with an extreme work level, defined as that associated with body signals values at least 3 SD to the right of the mean of body signal values. In yet another embodiment, the occurrence of simple or complex partial seizures with motor manifestations either positive *(e.g.,* muscle contractions) or negative *(e.g.,* lack of movements/motionless) may be determined, based on the correlation of body signal values and work level.

Returning to Figure 6, upon determination of at least the first work level at 620 and at least one work level excursion threshold at 640, the work level(s) may be compared at 650 to the threshold(s). If the work level is determined at 660 to be within the expected range or below a threshold(s), then a non-seizure occurrence may optionally be logged at 665 and flow may return to receiving at 610. A non-seizure occurrence may be further analyzed at a later time based upon additional data (*i.e.,* the manual declaration of a seizure by the patient or the declaration of a seizure based upon a different declaration systems) to dynamically tune at least one work level excursion threshold.

If the work level is determined at 660 to exceed the work level excursion threshold(s), then a seizure or other pathological state may be declared at 670. If desired, a verification function, such as a confirmation test, may be performed at 667 prior to the declaration at 670. In either event, after declaration at 670, a further action, *e.g.,* warning the patient, a caregiver, or a medical professional of the seizure or other pathological state; treating the pathological state; logging the pathological state's occurrence or a characteristic thereof; etc., may be performed at 680.

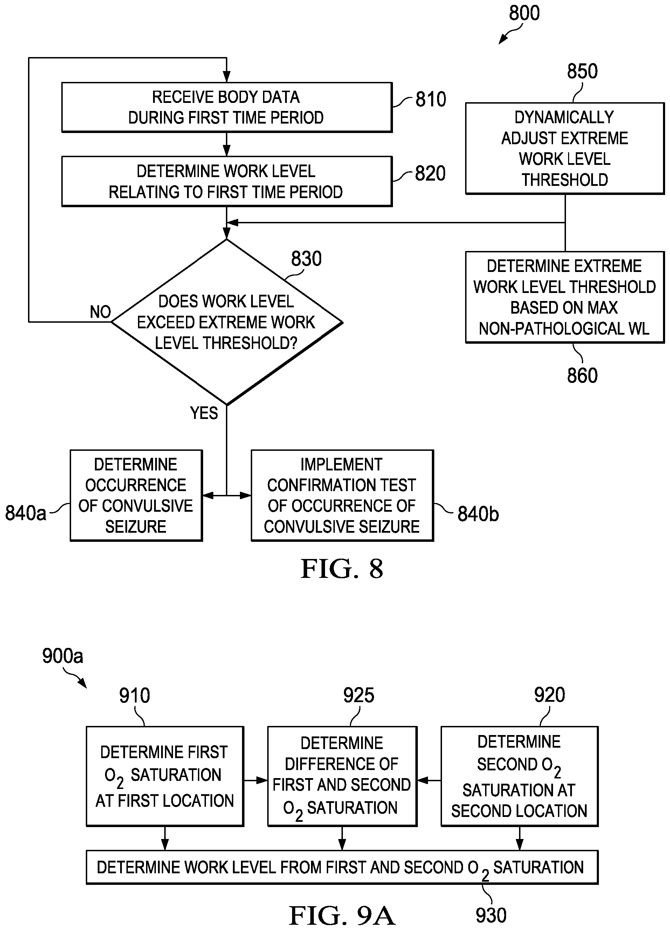

Figure 8 shows a flowchart representation of a method 800 of determining an occurrence of an epileptic convulsive seizure *(e.g.,* a primarily or secondarily generalized tonic seizure, a tonic-clonic seizure, or a clonic-tonic-clonic seizure, among others) in a patient. The method 800 may comprise receiving at 810 body data from a patient during a first time period. The body data may comprise at least one of kinetic data, oxygen saturation data, blood pH data, oxygen consumption data, glucose consumption data, respiratory rate, tidal volume, or end tidal CO₂ data. Kinetic data includes, but is not limited to, force, duration of contraction, rate of movement, amplitude of movement, velocity of movement, direction of movement, and duration of movement. Kinetic or autonomic data may be used to determine a patient's activity level, and this activity level may be in turn used to determine ("by proxy") work level. Oxygen saturation data, blood pH data, oxygen consumption data, glucose consumption data, respiratory rate, tidal volume, end tidal CO₂ data, or the like may be used to determine work level.

A work level relating to said first time period may be determined at 820 based at least partially upon said body data. If the work level is determined at 830 to exceed an extreme work level threshold, then one or more responsive actions may be performed 840, such as determining at 840a the occurrence of a convulsive seizure, or implementing at 840b a confirmation test to confirm the occurrence of a convulsive seizure, delivering a therapy, issuing a warning of the occurrence of the seizure, logging the date and time of occurrence of the seizure, determining a response to said therapy, determining an adverse effect of said therapy, determining a severity of the seizure, or determining if the seizure resulted in an injury to the patient, among others.

The confirmation test implemented at 840b may be a test based on cardiac data of said patient, a test based on respiratory data of said patient, a test of the patient's body movements, a test of the patient's responsiveness, or a test of the patient's awareness. Alternatively or in addition, in one embodiment, the confirmation test may involve a determination whether the patient's work level is incommensurate with a range expected for the time of day, week, month, or year, whether the patient's work level is incommensurate with the patient's activity level, or both.

In some embodiments, if desired and/or appropriate, the extreme work level threshold may be dynamically adjusted at 850 based at least in part on one or more of a time of day, environmental conditions (*e.g.,* time of day, ambient temperature, altitude, ambient humidity, etc.), a patient's body weight and height, a patient's body mass index, a patient's gender, a patient's age, an indicator of said patient's overall health, a patient's hydration status, or an indicator of said patient's overall fitness. For example, in a not-uncommon situation in the developing world, in a patient having a high fever under circumstances where the environmental temperature is also quite high, an intense complex partial seizure with a hypermotoric component may give rise to a detectable work level that, in cooler ambient conditions and/or for a non-febrile patient, would only be possible for a convulsive seizure. Alternatively or in addition, the extreme work level threshold may be determined at 860 based at least partially upon an historical indication of the patient's maximal non-pathological work level.

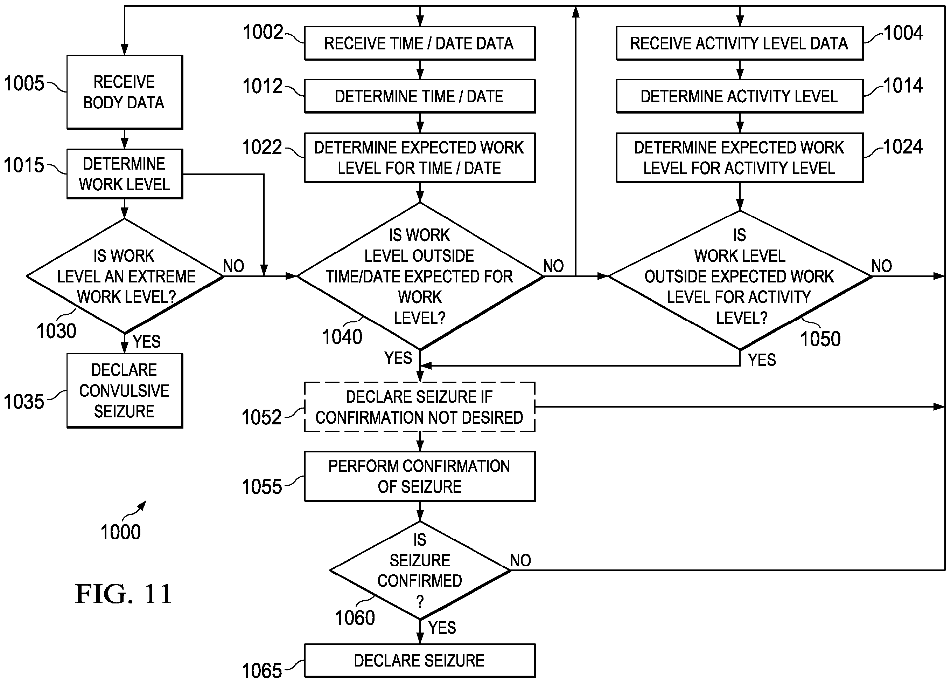

Turning now to Figure 11, this figure shows a method 1000 of detecting the occurrence of a seizure, according to some embodiments of the present disclosure. The method comprises at least receiving at 1005 body data relating to a patient's work level and determining at 1015 the patient's work level from the body data.

After determination at 1015 of the patient's work level, in one embodiment, it may be determined at 1030 whether the patient's work level is an extreme work level. If it is an extreme work level, a convulsive seizure may be declared at 1035. If it is not an extreme work level, flow may pass to either or both of determinations 1040 or 1050.

In other embodiments, after determination at 1015 of the patient's work level, flow may pass to either or both of determinations 1040 or 1050 and omit the determination at 1030.

In one embodiment, the method may comprise receiving at 1002 time/date data and determining at 1012 the time/date from the time/date data. In light of the time/date, an expected work level range may be determined at 1022. It may then be determined at 1040 if the work level (determined at 1015) is incommensurate with the work level range expected for the time/date. If it is incommensurate with the expected work level range, a seizure may be declared at 1052, if confirmation of the seizure is not desired, or a confirmation of the seizure may be performed at 1055.

For example, confirmation may be not desired if the therapy is safe, lacks side effects, and/or has not exceeded a maximum dosage in a current time window; if sensitivity, speed of detection, or both are desirable; if specificity is not particularly desirable; if the seizure has low intensity; or the like. For example, if a patient has partial seizures with secondary generalization (invariably associated with falls to the ground) that are abated if therapy is delivered within 5 sec of electrographic onset (while the patient is still aware and responsive), sensitivity and speed of detection may be maximized by omitting a confirmation of the declaration at 1052. Doing so may reduce the probability of serious injuries should the patient have a seizure.

On the other hand, confirmation may be desired if the therapy has unwelcome side effects; if specificity is desirable; if the drug supply *(e.g.,* of a therapeutic drug administered automatically from a drug reservoir) or the battery life *(e.g.,* of an electrical therapy device) is low. For example, if a patient with partial seizures with secondary generalization is lying in bed, and uses an anti-seizure therapy which is efficacious, but associated with intolerable side effects, specificity, not sensitivity or speed of detection, is paramount. Hence, a confirmation at 1055 of the seizure may be desirable. In one embodiment, the method may comprise receiving at 1004 activity level data regarding the patient and determining the patient's activity level at 1014. In light of the patient's activity level, an expected work level range may be determined at 1024. It may then be determined at 1050 if the work level (determined at 1015) is incommensurate with the work level range expected for the patient's activity level. If it is incommensurate with the expected work level range, a seizure may be declared at 1052, if confirmation is not desired, or a confirmation of a seizure may be performed at 1055. More specifically, if said observed work level is incommensurate with the activity level at the time of the measurement, but is within the physiological range, the epileptic seizure may be classified as partial and if the work level is above the upper range for non-ictal/physiological activity *(e.g.,* it reaches or is above the extreme threshold) the seizure may be classified as epileptic convulsive.
A confirmation of a seizure may be performed at 1055 using techniques described elsewhere herein, described in other patents or applications to Cyberonics, Inc., or Flint Hills Scientific, LLC or known to the person of ordinary skill in the art having the benefit of the present disclosure. After performing a confirmation at 1055, if it is determined at 1060 that a seizure is confirmed, a seizure may be declared at 1065, if the extreme work level has not been reached or exceed and if the work level is not commensurate with the activity level.
If a seizure is declared at 1052, or it is determined at 1060 that a seizure is not confirmed, then flow may return to receiving body data at 1005, receiving time/date data at 1002, and/or receiving activity level data at 1004. Flow may also return to one or more of elements 1005, 1002, or 1004 upon a "NO" result for the determinations at 1030, 1040, and/or 1050.
Turning now to Figure 9A, this figure shows a method 900a of determining a work level of a subject's body or a part thereof, which may be as follows. The method may comprise determining at 910 a first oxygen saturation, concentration, or pressure of the subject's blood at a first location. The method may comprise determining at 920 a second oxygen saturation, concentration, or pressure of the subject's blood at a second location. In some embodiments, the method may comprise determining at 925 a difference or a ratio between the first oxygen saturation, concentration, or pressure and the second oxygen saturation, concentration, or pressure. A difference determined at 925 can be determined simply by subtracting the second oxygen saturation, concentration, or pressure from the first, or vice versa and also encompasses determining the absolute value of the result of subtraction, should the result of subtraction be negative. A ratio determined at 925 can be determined simply by dividing the second oxygen saturation, concentration, or pressure by the first, or vice versa. The method may comprise determining at 930 the work level of the subject's body or part thereof based on the first and second oxygen saturation, concentration, or pressures and/or the difference between them.

In some embodiments, the first location is in a carotid artery; the second location is in a jugular vein; and the body part is the patient's brain. The first location and the second location may be located on a first side of the patient's body. This embodiment may be useful for detection of partial seizures, by measuring energy consumption of the brain. In other words, in some embodiments, the difference in oxygen saturation, concentration, or pressure between a carotid artery and an ipsilateral jugular vein may be used to detect the occurrence of partial seizures in patient with epilepsy.

It should be noted that, although the description so far has focused on gathering oxygen information of the anterior (e.g., carotid) circulation, the person of ordinary skill in the art would understand that the same method and procedure could be applied to the posterior circulation (vertebral arteries and branches thereof and posterior venous sinuses) to yield other information useful in determining a work level for the patient's brain, one or more parts thereof, the patient's body, or one or more parts thereof.

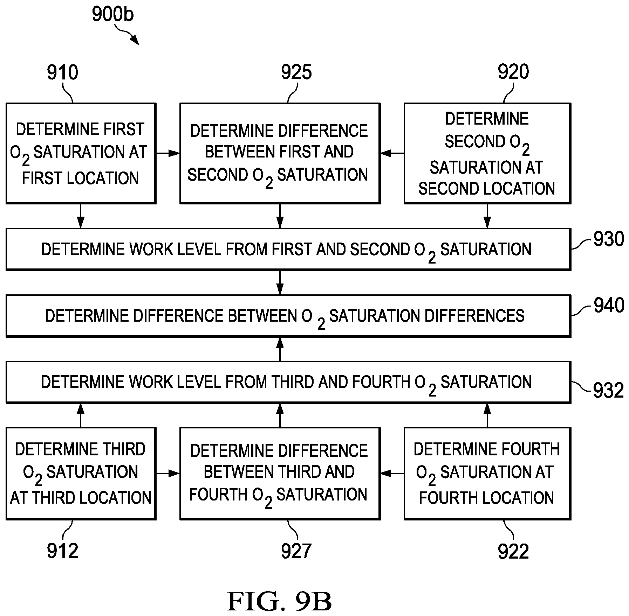

Turning to Figure 9B, which depicts a method 900b which has many like elements with Figure 9A that will not be further described, some embodiments may comprise one or more of determining at 912 a third oxygen saturation, concentration, or pressure of the subject's blood at a third location, determining at 922 a fourth oxygen saturation, concentration, or pressure of the subject's blood at a fourth location, determining at 927 a difference between the third oxygen saturation, concentration, or pressure and the fourth oxygen saturation, concentration, or pressure, or determining at 932 the work level of the subject's body or part thereof based on the third and fourth oxygen saturation, concentration, or pressures and/or the difference between them.

Thus, in some embodiments, the method 900b may comprise determining the work level of at least two regions of the subject's brain. In one embodiment, the at least two regions are the left and right hemispheres of the brain. In other embodiments, two or more of the at least two regions may be within the same hemisphere. The work level of the at least two regions of the subject's brain may be determined simultaneously, may be determined for overlapping time periods, or may be determined for non-overlapping time periods.

In embodiments comprising simultaneous bilateral measurements of oxygen consumption (*e.g.,* the left carotid artery may be the first location, the left jugular vein may be the second location, the right carotid artery may be the third location, and the right jugular vein may be the fourth location), such bilateral measurements may provide information regarding the unilaterality or bilaterality of seizures, the side of seizure origin, and the time of spread (if any) to the contralateral brain region. Bilateral measurements may also help distinguish partial from generalized and for the latter, primarily v. secondarily generalized. For seizures associated with movements of most or all body parts, oxygen consumption measurements may help distinguish epileptic from non-epileptic (psychogenic, "pseudo-seizures") seizures. Measurements of oxygen consumption of certain brain regions (*e.g.,* occipital lobes) not supplied by the carotid system, may be performed from posterior circulation system.

In some embodiments, the first location is in a blood vessel selected from a left ventricle, an aorta, a branch of the aorta *(e.g.,* carotid artery), or a sub-branch of the aorta (*e.g.,* middle cerebral artery); the second location is in a superior vena cava, an inferior vena cava, a right ventricle, a pulmonary artery, a jugular vein or a cerebral venous sinus; and the work level is determined of the subject's body.

In other embodiments the difference in oxygen saturation, concentration, or pressure between systemic or body arterial blood (which may be measured, for example, in the left ventricle) and systemic or body venous blood (which may be measured, for example, in the right ventricle) may be used to determine if the patient had an epileptic convulsion.

In some embodiments, information regarding work level may be determined by use of a single oxygen saturation, concentration, or pressure measurement at a single location, if compared to an appropriate control. In one embodiment, the oxygen saturation, concentration, or pressure measured at the first location may be compared with a negative control, *i.e.,* an oxygen saturation, concentration, or pressure measured at the first location during a verified non-seizure state. In another embodiment, the oxygen saturation, concentration, or pressure measured at the first location may be compared with a positive control, *i.e.,* an oxygen saturation, concentration, or pressure measured at the first location during a verified seizure state, such as partial seizure or a convulsive seizure. This embodiment may allow determinations of work level by use of only one oxygen saturation, concentration, or pressure sensor.

In yet another embodiment, a difference in the difference in arterial and venous blood oxygen saturation, concentration, or pressure as measured in a carotid artery and jugular vein compared to that measured in a right and left ventricles may be used to determine if the patient had a convulsion or a partial seizure.

In yet another embodiment, a bilateral difference between (i) the difference in oxygen saturation, concentration, or pressure measured in the left carotid artery and the left jugular vein and (ii) the difference in oxygen saturation, concentration, or pressure measured in the right carotid artery and the right jugular vein may be used to determine if the patient had a unilateral or a bilateral partial seizure. In other words, the difference between the oxygen saturation, concentration, or pressure differences may be determined (*e.g.,* at Figure 9B, 940). This embodiment may be particularly useful regarding seizures having an origin in the mesiotemporal lobe.

The methods depicted in Figures 6-9 and 11 and/or described above may be governed by instructions that are stored in a non-transitory computer readable storage medium and that are executed by, *e.g.,* a processor 217 of the medical device 200. Each of the operations shown in Figures 6-9 and 11 and/or described above may correspond to instructions stored in a non-transitory computer memory or computer readable storage medium. In various embodiments, the non-transitory computer readable storage medium includes a magnetic or optical disk storage device, solid state storage devices such as flash memory, or other non-volatile memory device or devices. The computer readable instructions stored on the non-transitory computer readable storage medium may be in source code, assembly language code, object code, or other instruction format that is interpreted and/or executable by one or more processors.

## Claims

1. A medical device (200), comprising:
a body data module (175) configured to receive body data;
a work level data module (150) configured to determine a first patient work level during a first time period, and a second patient work level during a second time period, wherein the work level refers to the energy consumption of the patient's body or a part thereof;
an activity level module (162) configured to determine an activity level of the patient based at least in part on one or more of the body data, a time of day, or an activity level history of the patient,
a work level excursion module (180) configured to:
determine whether said second work level exceeds said first work level by an amount greater than a first work level excursion threshold;
detect a work level excursion, in particular, a pathological work level excursion, based upon a determination that said second work level exceeds said first work level by an amount greater than said first work level excursion threshold, wherein said work level excursion threshold is based at least in part on one or more of a time of day, an environmental condition, an indicator of said patient's hydration status, said patient's body weight and height, said patient's body mass index, said patient's gender, or said patient's age; and
detect a pathological state based upon that said second work level does not exceed said first work level by an amount greater than said first work level excursion threshold and a determination that the second work level is outside a work level range expected for the patient's activity level; and
a controller (110) to perform at least one of warning of said work level excursion, providing a therapy for said work level excursion, or logging an occurrence of said work level excursion in response to detecting said work level excursion.

2. The medical device (200) of claim 1, wherein the first time period is at a first time of day on a current day and the second time period is at the first time of day on at least one prior day and/or first time period and the second time period are contiguous, partially overlapping, or fully overlapping; and/or
the first patient work level and the second patient work level are each a work level magnitude or a work level rate of change.

3. The medical device (200) of claim 1 or 2, further comprising:
at least one sensor (112), each configured to collect at least one body signal from a patient relating to a work level of said patient, wherein said at least one sensor, in particular, comprises at least one of:
an autonomic data acquisition unit (260) configured to acquire at least one of heart beat data, blood pressure data, respiration data, or blood gas data;
a neurological data acquisition unit (266) configured to acquire kinetic data selected from the group consisting of accelerometer data and inclinometer data.
an endocrine data acquisition unit (273) configured to acquire endocrine data,
a metabolic data acquisition unit (274) configured to acquire metabolic data;
a tissue stress marker data acquisition unit (275) configured to acquire tissue stress marker data; or
a physical fitness data acquisition unit (276) configured to acquire data relating to the patient's physical fitness.

4. The medical device (200) of one of the claims 1 to 3, wherein said medical device (200) is configured to confirm said pathological state based on at least one of cardiac data of said patient, kinetic data of said patient, the patient's responsiveness, or a patient's awareness.

5. The medical device (200) of one of the claim 1 to 4, wherein said work level data module (150) comprises:
a body data analysis module (310) to determine a value relating to at least one of a cardiac data, blood pressure data, respiration data, blood gas data, patient kinetic data, responsiveness data, endocrine data, metabolic data, or tissue stress marker data; and a work level determination unit (320) to determine said first and second patient work levels based upon at least one of said cardiac data, blood pressure data, respiration data, blood gas data, patient kinetic data, responsiveness data, endocrine data, metabolic data, or tissue stress marker data.

6. The medical device (200) of claim 5, further comprising a work level excursion module (180) configured to determine the work level excursion threshold based on at least one of said body data, a time of day, or a patient state.

7. The medical device (200) of claim 5 or 6, further comprising:
a background activity time period module (540) configured to provide data relating to historic patient activity in relation to time of day;
a body data activity correlation unit (510) configured to correlate said body data to at least one of a plurality of candidate states based upon said data relating to historic patient activity; and
a patient input unit configured to receive an input from said patient.

8. The medical device (200) of one of the claims 1 to 7, further comprising:
a warning unit (192) to provide a warning in response to a determine that a work level excursion has occurred;
a therapy unit (194) configured to provide a therapy in response to a determine that a work level excursion has occurred; and
a logging unit (196) to log at least one of an occurrence or a severity of said work level excursion, wherein logging comprises storing to memory one or more of:
at least one of a date, a time, or a severity of said detected work level excursion;
a type of therapy delivered to treat said work level excursion; or
at least one effect of a therapy delivered to treat the work level excursion.

## Patentansprüche

1. Eine medizinische Vorrichtung (200), umfassend:
ein Körperdatenmodul (175), das konfiguriert ist, Körperdaten zu empfangen;
ein Arbeitsniveau-Datenmodul (150), das so konfiguriert ist, dass es ein erstes Patienten-Arbeitsniveau während einer ersten Zeitspanne und ein zweites Patienten-Arbeitsniveau während einer zweiten Zeitspanne bestimmt, wobei sich das Arbeitsniveau auf den Energieverbrauch des Körpers des Patienten oder eines Teils davon bezieht;
ein Aktivitätsniveau-Modul (162), das so konfiguriert ist, dass es ein Aktivitätsniveau des Patienten zumindest teilweise auf der Grundlage der Körperdaten und/oder einer Tageszeit und/oder einer Aktivitätsniveau-Historie des Patienten bestimmt;
ein Arbeitsniveau-Abweichungsmodul (180), das so konfiguriert ist, dass es:
bestimmt, ob das zweite Arbeitsniveau das erste Arbeitsniveau um einen Betrag überschreitet, der größer ist als ein erster Arbeitsniveau-Abweichungsschwellenwert;
eine Arbeitsniveau-Abweichung erkennt, insbesondere eine pathologische Arbeitsniveau-Abweichung, auf der Grundlage einer Bestimmung, dass das zweite Arbeitsniveau das erste Arbeitsniveau um einen Betrag überschreitet, der größer ist als der erste Arbeitsniveau-Abweichungsschwellenwert, wobei der Arbeitsniveau-Abweichungsschwellenwert zumindest teilweise auf einer Tageszeit und/oder einer Umgebungsbedingung und/oder einem Indikatorfür den Hydratationsstatus des Patienten und/oder dem Körpergewicht und der Körpergröße des Patienten und/oder dem Body-Mass-Index des Patienten und/oder dem Geschlecht des Patienten und/oder dem Alter des Patienten basiert; und
einen pathologischen Zustand erkennt, basierend auf der Grundlage, dass das zweite Arbeitsniveau das erste Arbeitsniveau nicht um einen Betrag übersteigt, der größer ist als der erste Arbeitsniveau-Abweichungsschwellenwert, und einer Bestimmung, dass das zweite Arbeitsniveau außerhalb eines für das Aktivitätsniveau des Patienten erwarteten Arbeitsniveaubereichs liegt; und
eine Steuereinheit (110) zum Ausführen einer Warnung vor der Arbeitsniveau-Abweichung und/oder eines Bereitstellens einer Therapie für die Arbeitsniveau-Abweichung und/oder eines Protokollierens eines Auftretens der Arbeitsniveau-Abweichung als Reaktion auf das Erkennen der Arbeitsniveau-Abweichung.

2. Die medizinische Vorrichtung (200) nach Patentanspruch 1, wobei die erste Zeitspanne zu einer ersten Tageszeit an einem aktuellen Tag liegt und die zweite Zeitspanne zu der ersten Tageszeit an mindestens einem vorherigen Tag liegt und/oder die erste Zeitspanne und die zweite Zeitspanne aneinander angrenzen, sich teilweise überschneiden oder sich vollständig überschneiden; und/oder
das erste Patienten-Arbeitsniveau und das zweite Patienten-Arbeitsniveau sind jeweils eine Arbeitsniveau-Größe oder eine Arbeitsniveau-Änderungsrate.

3. Die medizinische Vorrichtung (200) nach Patentanspruch 1 oder 2, ferner umfassend:
mindestens einen Sensor (112), der jeweils so konfiguriert ist, dass er mindestens ein Körpersignal von einem Patienten erfasst, das sich auf ein Arbeitsniveau des Patienten bezieht, wobei der mindestens eine Sensor insbesondere umfasst:
eine autonome Datenerfassungseinheit (260), die so konfiguriert ist, dass sie Herzschlagdaten und/oder Blutdruckdaten und/oder Atmungsdaten und/oder Blutgasdaten erfasst; und/oder
eine Erfassungseinheit für neurologische Daten (266), die so konfiguriert ist, dass sie kinetische Daten erfasst, die aus der Gruppe bestehend aus Beschleunigungsmesserdaten und Neigungsmesserdaten ausgewählt werden; und/oder
eine Erfassungseinheit für endokrine Daten (273), die konfiguriert ist, endokrine Daten zu erfassen;
eine Erfassungseinheit für metabolische Daten (274), die konfiguriert ist, metabolische Daten zu erfassen; und/oder
eine Erfassungseinheit für Gewebespannungsmarkerdaten (275), die konfiguriert ist, Gewebespannungsmarkerdaten zu erfassen; und/oder
eine Erfassungseinheit für körperliche Fitness-Daten (276), die konfiguriert ist, Daten zu erfassen, die sich auf die körperliche Fitness des Patienten beziehen.

4. Die medizinische Vorrichtung (200) nach einem der Patentansprüche 1 bis 3, wobei die medizinische Vorrichtung (200) so konfiguriert ist, dass sie den pathologischen Zustand auf der Grundlage von Herzdaten des Patienten und/oder kinetischen Daten des Patienten und/oder der Reaktionsfähigkeit des Patienten und/oder des Bewusstseins des Patienten bestätigt.

5. Die medizinische Vorrichtung (200) nach einem der Patentansprüche 1 bis 4, wobei das Arbeitsniveau-Datenmodul (150) umfasst:
ein Körperdaten-Analysemodul (310), um einen Wert zu bestimmen, der sich auf Herzdaten und/oder Blutdruckdaten und/oder Atmungsdaten und/oder Blutgasdaten und/oder kinetische Daten des Patienten und/oder Reaktionsdaten und/oder endokrine Daten und/oder Stoffwechseldaten und/oder Gewebespannungsmarkerdaten bezieht; und
eine Arbeitsniveau-Bestimmungseinheit (320) zum Bestimmen des ersten und zweiten Patienten-Arbeitsniveaus auf der Grundlage von Herzdaten und/oder Blutdruckdaten und/oder Atmungsdaten und/oder Blutgasdaten und/oder kinetische Daten des Patienten und/oder Reaktionsdaten und/oder endokrine Daten und/oder metabolische Daten und/oder Gewebespannungsmarkerdaten.

6. Die medizinische Vorrichtung (200) nach Patentanspruch 5, ferner umfassend ein Arbeitsniveau-Abweichungsmodul (180), das konfiguriert ist, den Arbeitsniveau-Abweichungsschwellenwert auf der Grundlage der Körperdaten und/oder der Tageszeit und/oder des Patientenzustands zu bestimmen.

7. Die medizinische Vorrichtung (200) nach Patentanspruch 5 oder 6, ferner umfassend:
ein Hintergrundaktivitäts-Zeitspanne-Modul (540), das konfiguriert ist, Daten, die sich auf historische Patientenaktivität beziehen, in Bezug auf die Tageszeit zu liefern;
eine Körperdaten-Aktivitätskorrelationseinheit (510), die so konfiguriert ist, dass sie die Körperdaten mit wenigstens einem aus einer Vielzahl von möglichen Zuständen auf der Grundlage der Daten, die sich auf historische Patientenaktivität beziehen, korreliert; und
eine Patienteneingabeeinheit, die konfiguriert ist, eine Eingabe von dem Patienten zu empfangen.

8. Die medizinische Vorrichtung (200) nach einem der Patentansprüche 1 bis 7, ferner umfassend:
eine Warneinheit (192), die eine Warnung ausgibt, wenn festgestellt wird, dass eine Arbeitsniveau-Abweichung stattgefunden hat;
eine Therapieeinheit (194), die konfiguriert ist, eine Therapie in Reaktion auf die Feststellung, dass eine Arbeitsniveau-Abweichung aufgetreten ist, bereitzustellen; und
eine Protokollierungseinheit (196) zum Protokollieren eines Auftretens und/oder einer Schwere der Arbeitsniveau-Abweichung, wobei das Protokollieren das Speichern in einem Speicher umfasst:
Datum und/oder Uhrzeit und/oder Schwere der erkannten Arbeitsniveau-Abweichung; und/oder
eine Art von Therapie, die zur Behandlung der Arbeitsniveau-Abweichung durchgeführt wird; oder
wenigstens eine Wirkung einer Therapie, die zur Behandlung der Arbeitsniveau-Abweichung durchgeführt wird.

## Revendications

1. Dispositif médical (200), comprenant:
un module de données corporelles (175) configuré pour recevoir des données corporelles;
un module de données sur le niveau de travail (150) configuré pour déterminer un premier niveau de travail d'un patient au cours d'une période d'évaluation d'un niveau de travail d'un patient;
une première période de temps, et un second niveau de travail du patient pendant une seconde période de temps, dans lequel le niveau de travail se réfère à la consommation d'énergie du corps du patient ou d'une partie de celui-ci;
un module de niveau d'activité (162) configuré pour déterminer un niveau d'activité du patient sur la base d'au moins une partie des données corporelles, d'une heure de la journée ou d'un historique de niveau d'activité du patient,
un module d'excursion de niveau de travail (180) configuré pour:
déterminer si ledit second niveau de travail dépasse ledit premier niveau de travail d'une quantité supérieure à un seuil d'excursion du premier niveau de travail;
détecter une excursion du niveau de travail, en particulier une excursion pathologique du niveau de travail, sur la base d'une détermination du fait que ledit second niveau de travail dépasse ledit premier niveau de travail d'une quantité supérieure audit premier seuil d'excursion du niveau de travail, dans lequel ledit seuil d'excursion du niveau de travail est fondé au moins en partie sur un ou plusieurs éléments parmi une heure de la journée, une condition environnementale, un indicateur de l'état d'hydratation dudit patient, le poids et la taille dudit patient, l'indice de masse corporelle dudit patient, le sexe dudit patient ou l'âge dudit patient; et
détecter un état pathologique sur la base du fait que ledit second niveau de travail ne dépasse pas ledit premier niveau de travail d'une quantité supérieure audit premier seuil d'excursion de niveau de travail et d'une détermination que le second niveau de travail est en dehors d'une plage de niveaux de travail attendue pour le niveau d'activité du patient; et
un dispositif de commande (110) pour effectuer au moins l'une des opérations suivantes : avertissement de ladite excursion de niveau de travail, fourniture d'une thérapie pour ladite excursion de niveau de travail, ou enregistrement d'une occurrence de ladite excursion de niveau de travail en réponse à la détection de ladite excursion de niveau de travail.

2. Dispositif médical (200) selon la revendication 1, dans lequel la première période de temps est à un premier moment de la journée sur un jour actuel et la seconde période de temps est au premier moment de la journée sur au moins un jour antérieur et/ou la première période de temps et la seconde période de temps sont contiguës, se chevauchent partiellement, ou se chevauchent complètement ; et/ou
le premier niveau de travail du patient et le second niveau de travail du patient sont chacun une grandeur de niveau de travail ou un taux de changement de niveau de travail.

3. Dispositif médical (200) selon les revendications 1 ou 2, comprenant en outre:
au moins un capteur (112), chacun configuré pour collecter au moins un signal corporel d'un patient relatif à un niveau de travail dudit patient, dans lequel ledit au moins un capteur, en particulier, comprend au moins l'un des éléments suivants:
une unité d'acquisition de données autonomes (260) configurée pour acquérir au moins l'une des données de battement cardiaque, des données de pression sanguine, des données de respiration ou des données de gaz sanguin;
une unité d'acquisition de données neurologiques (266) configurée pour acquérir des données cinétiques sélectionnées dans le groupe constitué de données d'accéléromètre et de données d'inclinomètre;
une unité d'acquisition de données endocrines (273) configurée pour acquérir des données endocrines, une unité d'acquisition de données métaboliques (274) configurée pour acquérir des données métaboliques;
une unité d'acquisition de données de marqueur de contrainte de tissu (275) configurée pour acquérir des données de marqueur de contrainte de tissu; ou
une unité d'acquisition de données de condition physique (276) configurée pour acquérir des données relatives à la condition physique du patient.

4. Dispositif médical (200) selon l'une des revendications 1 à 3, dans lequel ledit dispositif médical (200) est configuré pour confirmer ledit état pathologique sur la base d'au moins l'une des données cardiaques dudit patient, des données cinétiques dudit patient, de la réactivité du patient, ou de la conscience du patient.

5. Dispositif médical (200) selon l'une des revendications 1 à 4, dans lequel ledit module de données de niveau de travail (150) comprend:
un module d'analyse des données corporelles (310) pour déterminer une valeur relative à au moins une des données cardiaques, des données de pression sanguine, des données de respiration, des données de gaz sanguin, des données cinétiques du patient, des données de réactivité, des données endocriniennes, des données métaboliques ou des données de marqueur de stress tissulaire; et
une unité de détermination de niveau de travail (320) pour déterminer lesdits premier et second niveaux de travail du patient sur la base d'au moins l'une desdites données cardiaques, données de pression sanguine, données de respiration, données de gaz sanguin, données cinétiques du patient, données de réactivité, données endocriniennes, données métaboliques ou données de marqueur de stress tissulaire.

6. Dispositif médical (200) selon la revendication 5, comprenant en outre un module d'excursion de niveau de travail (180) configuré pour déterminer le seuil d'excursion de niveau de travail sur la base d'au moins l'une desdites données corporelles, d'une heure de la journée ou d'un état du patient.

7. Dispositif médical (200) selon les revendications 5 ou 6, comprenant en outre:
un module de période d'activité de fond (540) configuré pour fournir des données relatives à l'activité historique du patient en fonction de l'heure de la journée;
une unité de corrélation d'activité de données corporelles (510) configurée pour corréler lesdites données corporelles à au moins un d'une pluralité d'états candidats sur la base desdites données relatives à l'activité historique du patient; et
une unité d'entrée de patient configurée pour recevoir une entrée dudit patient.

8. Dispositif médical (200) selon l'une des revendications 1 à 7, comprenant en outre:
une unité d'avertissement (192) pour fournir un avertissement en réponse à une détermination qu'une excursion de niveau de travail s'est produite;
une unité de thérapie (194) configurée pour fournir une thérapie en réponse à une détermination qu'une excursion du niveau de travail s'est produite; et
une unité d'enregistrement (196) pour enregistrer au moins l'une d'une occurrence ou d'une gravité de ladite excursion de niveau de travail, dans laquelle l'enregistrement comprend le stockage en mémoire d'un ou plusieurs des éléments suivants:
au moins un élément parmi une date, une heure ou une gravité de ladite excursion de niveau de travail détectée;
un type de thérapie délivré pour traiter ladite excursion du niveau de travail; ou
au moins un effet d'une thérapie délivrée pour traiter l'excursion du niveau de travail.
